# EUROPEAN PATENT SPECIFICATION

(11) **EP 2 566 558 B1**
(45) Date of publication and mention of the grant of the patent: **17.04.2019**
(21) Application number: 11719954.7
(22) Date of filing: 04.05.2011
(51) Int. Cl.: A61M 25/00, A61M 25/09, A61M 25/01, A61B 17/22

(54) **INTRALUMINAL TREATMENT APPARATUS, WIRE GUIDE AND TREATMENT FLUID DELIVERY METHOD**
INTRALUMINALE BEHANDLUNGSVORRICHTUNG, DRAHTFÜHRUNG UND VERFAHREN ZUR FREISETZUNG EINER BEHANDLUNGSFLÜSSIGKEIT
APPAREIL DE TRAITEMENT INTRALUMINAL, FIL-GUIDE ET PROCÉDÉ D'ADMINISTRATION DE FLUIDE DE TRAITEMENT

(30) Priority: 05.05.2010 US 331546 P
(43) Date of publication of application: 13.03.2013
(73) Proprietor: Cook Medical Technologies LLC, Bloomington, IN 47404 (US)
(72) Inventor: RANDOLPH, James, R., Bedford, IN 47421 (US); OSBORNE, Thomas, A., Bloomington IN 47401 (US)
(74) Representative: Garratt, Peter Douglas
(86) International application number: PCT/US2011/035115
(87) International publication number: WO 2011/140177

(56) References cited:
- EP-A1- 0 963 764
- EP-A1- 1 013 301
- WO-A1-03/033050
- US-A- 5 709 874
- US-B1- 6 402 736

## Description

### Technical Field

The present disclosure relates generally to mechanisms for intraluminal treatment of a patient, and relates more particularly to adjusting a flow managing mechanism within a body lumen from a first configuration defined by a dominant shape memory property to a second configuration defined by a subordinate shape memory property.

### Background

A wide variety of medical procedures involve the supplying of a treatment fluid into a body lumen of a patient. Peripheral intervention techniques in the human cardiovascular system represent one class of medical procedures where supplying of a treatment fluid is well known for a variety of purposes. For instance, thrombolytic agents are commonly delivered by way of infusion into a vein or artery for the purpose of breaking up and/or dissolving clot material. Another peripheral intervention procedure involves supplying a chemotherapeutic agent which may be carried by blood flow to a treatment site of interest such as a tumor. Peripheral intervention commonly entails percutaneous access to a patient's cardiovascular system. A great many different devices and techniques have been developed over the years for percutaneously accessing treatment sites, and supplying treatment fluids such as the thrombolytic and chemotherapeutic agents mentioned above. Some of these strategies have met with great success in certain treatment contexts, but improvements could be made in others.

Many body tissues are relatively insensitive to the effects of certain treatment fluids such as dyes, saline, and others. Other treatment fluids may be relatively toxic regardless of tissue type. Such toxicity is not entirely unintended, as in the case of chemotherapeutic agents. Chemotherapeutics are typically designed to kill tumor cells, but can often also damage healthy tissue. While certain thrombolytic agents might not necessarily be considered "toxic," they can have deleterious effects on various parts of the body such as by inducing bleeding. The desire to avoid overuse of certain treatment fluids, and avoid administration of such fluids outside of target locations, will thus be readily apparent. Such overuse or extraneous administration, however, remains relatively common due to at least in part to difficulty in accessing certain parts of the body, and difficulty in controlling flow of treatment fluids in vivo.

For example, the location, structure or other properties of a target site for initiating delivery of a treatment fluid within a patient's vasculature may present challenges to delivering the right amount of treatment fluid, to the right place, while avoiding delivering the treatment fluid to unintended parts of the body. In the case of certain chemotherapy procedures, an advantageous location for initiating delivery of a treatment fluid may lie upstream of a branching vascular structure, where one branch supplies blood to a target treatment site such as a tumor, and another branch supplies blood to healthy tissue or a location otherwise desirable to protect from exposure to the treatment fluid.

A technique known as embolization is commonly used to block or limit blood flow to certain areas of a patient's vasculature or other parts of the body. One conventional embolization technique involves the placement of a device within a vascular lumen to be blocked or flow-limited, where the device includes attached fibrous material to promote the formation of clot material. It has been observed that deployment of such devices is often followed almost immediately by formation of a clot. This general strategy can indeed prevent exposure of downstream tissue to a chemotherapeutic or other treatment agent since the clot effectively stops blood flow in the subject vascular lumen. This approach has a number of disadvantages, however, principle among them being the need to typically leave the embolization device and clot in place within the vascular lumen. Not only is a foreign object left within the patient, thrombus material is also formed, and permanent changes in blood flow patterns in response to the blocked vascular lumen may result.

Other procedures are known in which an embolization mechanism is removed from the patient after the treatment procedure concludes. Such mechanisms may include inflatable balloons and the like which are uninflated and removed when it is no longer desirable to restrict blood flow. Each of the above general types of embolization technique suffer from drawbacks, and in particular may be poorly suited to embolization procedures in which it is necessary to restrict blood flow through an especially small vascular lumen. As a result, clinicians may choose to forego embolization during certain procedures, having determined that the potential consequences of overuse or supplying toxic fluid where it is not needed are outweighed by the benefits of treating the patient. Document EP 1 013 301 A1 discloses a tube shaped catheter body and a shaping member provided at least at the front end portion of the catheter main body such that the shaping member can impart a winding shape to the front end portion of the catheter and a cavity portion formed at the inner side of the shaping member.

### Summary of the Disclosure

According to the invention an intraluminal treatment apparatus includes an elongate catheter body having a profiled catheter tip as defined in claim 1.

In another aspect, not according to the invention, a wire guide includes an elongate outer wire body defining a longitudinal axis extending between a proximal outer body segment and a distal outer body segment adjoining a distal outer body tip. The distal outer body segment has a subordinate shape memory property and includes a fixed primary shape, a fixed secondary shape, and a mutable tertiary shape. The wire guide further includes an elongate inner wire body positioned within the outer wire body and including a proximal inner body segment and a distal inner body segment adjoining a distal inner body tip. The inner wire body is slidable relative to the outer wire body between a first axial location and a second axial location, and includes a dominant shape memory property. The distal outer body segment includes a first wire thickness, and the distal inner body segment includes a second wire thickness which is greater than the first wire thickness. The wire guide includes a low profile configuration in which the inner wire body is positioned at the first axial location and the dominant shaped memory property defines the tertiary shape, and an expanded configuration in which the inner wire body is positioned at the second axial location and the subordinate shape memory property defines the tertiary shape.

In still another aspect, not according to the invention, a method of delivering a treatment fluid to a body lumen includes adjusting a flow managing mechanism between a lumen access configuration and a deployed configuration within a first body lumen. The flow managing mechanism includes a helical flow control component having a subordinate shape memory property, and a non-helical shape control component having a dominant shape memory property and being positioned within the helical flow control component. The method further includes supplying a treatment fluid to a second body lumen in fluid communication with the first body lumen. The method still further includes limiting supplying the treatment fluid to the first body lumen at least in part by occluding the first body lumen with the flow managing mechanism in the deployed configuration. Adjusting the flow managing mechanism further includes sliding the shape control component relative to the flow control component between a first location at which the dominant shape memory property imparts the lumen access configuration to the flow managing mechanism, and a second location at which the subordinate shape memory property imparts the deployed configuration.

### Brief Description of the Drawings

Figure 1 is a partially sectioned diagrammatic view of a packaged intraluminal treatment apparatus, according to one embodiment;
Figure 2 is a sectioned side diagrammatic view of a flow managing mechanism, according to one embodiment;
Figure 3 is a side diagrammatic view of a flow managing mechanism according to one embodiment, in a first deployed configuration;
Figure 4 is a side diagrammatic view of the flow managing mechanism of Figure 3, in a second deployed configuration;
Figure 5 is a partially sectioned side diagrammatic view of the flow managing mechanism of Figures 3 and 4, in the second deployed configuration;
Figure 6 is a side diagrammatic view of a flow managing mechanism in a deployed configuration, according to another embodiment;
Figure 7 is a side diagrammatic view of a flow managing mechanism in a deployed configuration, according to yet another embodiment;
Figure 8 is a pictorial view of an intraluminal treatment apparatus positioned within a patient, at one stage of an intraluminal treatment procedure;
Figure 9 is a pictorial view of the intraluminal treatment apparatus of Figure 8 positioned within a patient, at another stage of an intraluminal treatment procedure; and
Figure 10 is a pictorial view of an intraluminal treatment apparatus at one stage of an intraluminal treatment procedure, according to another embodiment.

### Detailed Description

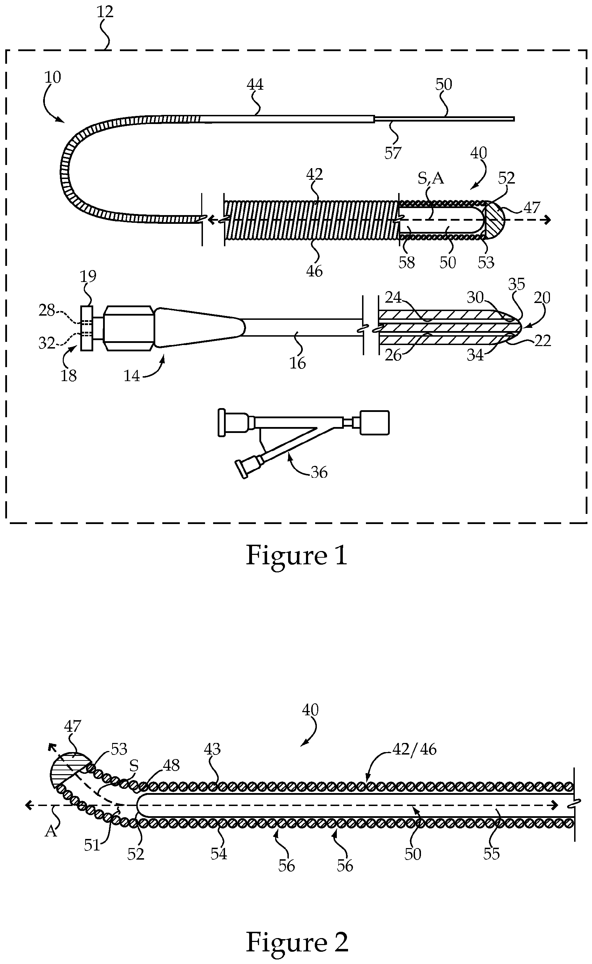

Referring to Figure 1, there is shown an intraluminal treatment apparatus 10 according to one embodiment. Apparatus 10 may include a plurality of different components, positioned within a sterile package 12, and having an assembly configuration with one another for performing intraluminal treatment procedures, as further described herein. Sterile package 12 may include a sealed, peel-open pouch in one embodiment. Apparatus 10 may include a fluid supply mechanism 14 for supplying a treatment fluid to a body lumen of a patient, and a flow managing mechanism 40 for controlling a flow of fluid within the body lumen. An adapter mechanism 36 such as a Y-fitting may also be provided. Additional components (not shown) such as a tube protector coil or a conventional wire guide may also be included in sterile package 12. As will be further apparent from the following description, apparatus 10 may be uniquely adapted for controlling and managing fluid flow within a body lumen of a patient by varying certain shape attributes thereof.

Mechanism 14 may include an elongate catheter body 16 having a proximal catheter end 18, and a distal catheter end 20. A fitting/manifold 19 may be located at proximal catheter end 18, and configured to connect with an adapter mechanism such as Y-fitting 36 in a conventional manner. Distal catheter end 20 may include a profiled catheter tip 22, having a narrowing taper in a proximal to distal direction, and defining a fluid outlet 35 for supplying a treatment fluid to a body lumen of a patient. Other profiled, atraumatic catheter tip configurations such as a hemispheric shape might also be used. Elongate catheter body 16 may further define at least one longitudinally extending passage including, for example, a first passage 24 and a second passage 26. First passage 24 may include a fluid supply lumen having a proximal passage end 28 opening at proximal catheter end 18, and a distal passage end 30 fluidly connecting with outlet 35 and opening in catheter tip 22. Passage 26 may include a longitudinally extending device lumen having a proximal passage end 32 opening at proximal catheter end 18, and a distal passage end 34 opening in catheter tip 22. Each of passages 24 and 26 may include a passage length between their respective passage ends.

Flow managing mechanism 40 may include an elongate flow control component 42 having a proximal component segment 44, and a distal component segment 46. Mechanism 40 may further include an elongate shape control component 50 positioned within flow control component 42 and having a proximal component segment 57 and a distal component segment 58. Shape control component 50 may be slidable relative to flow control component 42. Flow control component 42 may include an outer wire body, further described herein, and segments 44 and 46 may include a proximal outer body segment and a distal outer body segment. Shape control component 50 may include an inner wire body, further described herein, and segments 57 and 58 may include proximal and distal inner body segments. Flow control component 42 may define a first longitudinal axis S, whereas shape control component 50 may define a second longitudinal axis A. In Figure 1, flow control component 42 and shape control component 50 are arranged coaxially and, hence, longitudinal axes S and A are illustrated as overlapping. Sliding shape control component 50 relative to flow control component 42 can change a shape of flow managing mechanism 40 such that longitudinal axes S and A become at least partially non-overlapping, for purposes which will be apparent from the following description.

Elongate flow control component 42 may include a distal tip 47 adjoining segment 46 and having a tip inner surface 53. Shape control component 50 may include a distal tip 52 adjoining segment 58, which may be rounded to enable shape control component 50 to readily slide in either a proximal to distal or a distal to proximal direction within flow control component 42. Stiffness of component 50 in a region adjacent distal tip 52 might also be relatively lower than other regions of component 50, for similar purposes. Each of components 42 and 50 may include an axial component length between their respective terminal ends which is greater than the passage length(s) of passages 24 and 25. In Figure 1, shape control component 50 is shown at a first axial location within flow control component 42 at which distal tip 52 abuts or is adjacent to tip inner surface 53. When shape control component 50 is positioned at the first axial location within flow control component 42, approximately as shown in Figure 1, flow managing mechanism 40 may be understood to be in a low profile configuration which includes a lumen access configuration of mechanism 40. When shape control component 50 is positioned at a second axial location, flow managing mechanism may be understood to be in an expanded configuration which includes a deployed configuration of mechanism 40. The low profile configuration facilitates accessing a body lumen in a patient with flow managing mechanism 40, whereas the expanded configuration enables managing or controlling fluid flow as further described herein.

Referring also to Figure 2, there is shown a sectioned side diagrammatic view of a portion of flow managing mechanism 40. In one embodiment, flow managing mechanism 40 may be configured to function as a wire guide useful for performing, or assisting in performing a variety of intraluminal treatment procedures such as percutaneous treatment procedures. To this end, flow control component 42 may include an elongate outer wire body 54, and shape control component 50 may include an elongate inner wire body 55 slidable relative to outer wire body 54. Outer wire body 54 may also include a helical wire body having a plurality of windings 56, and inner wire body 55 may include a non-helical wire body coaxial with the helical wire body. Windings 56 may include an exposed outer surface 43 of flow control component 42, and an inner surface 48 defining a control lumen 51, receiving shape control component 50 therein.

It will be recalled that sliding shape control component 50 within flow control component 42 enables a shape of flow control component 42, and thus a configuration of flow managing mechanism 40, to be adjusted between a low profile configuration and an expanded configuration. Flow control component 42 may include a fixed primary shape, a fixed secondary shape, and a mutable tertiary shape. These and other geometric attributes of mechanism 40 are further described below. The mutable tertiary shape may be the main subject of shape adjustment according to the present disclosure. It will also be recalled that shape control component 50 is shown at a first axial location within flow control component 42 in Figure 1. The axial location of shape control component 50 shown in Figure 1 may be understood as a distally advanced location. In Figure 2, shape control component 50 has been slid in a distal to proximal direction within flow control component 42 to a second axial location. The axial location of shape control component 50 depicted in Figure 2 may be understood as a retracted position. It may also be noted from Figure 2 that a shape of flow control component 42 has changed from the shape depicted in Figure 1. Longitudinal axes A and S are partially overlapping in the configuration shown in Figure 2, but begin to diverge from one another near distal tip 47.

As mentioned above, the configuration of flow managing mechanism 40 shown in Figure 1 includes a low profile configuration for accessing a body lumen, for example. In the low profile configuration, outer wire body 54 may be understood to be radially condensed relative to longitudinal axis A. As used herein, the term "radially condensed" means that a radius of a circle having a center within longitudinal axis A and a perimeter tangent to a radially outermost point of flow control component 42, e.g. tangent to radially outermost points of surface 43, is relatively small. In the configuration shown in Figure 2, outer wire body 54 may be understood to be radially expanded relative to longitudinal axis A. "Radially expanded" means that the radius described above is relatively large. The change in shape in flow managing mechanism 40 which is evident from comparing Figure 1 with Figure 2, and much more dramatic changes in shape as further described herein, result at least in part from differing shape memory properties of shape control component 50 versus flow control component 42.

To this end, flow control component 42 may include a subordinate shape memory property and shape control component 50 may include a dominant shape memory property. Flow control component 42 may assume a shape determined by the dominant shape memory property under certain conditions, and may assume a shape determined by the subordinate shape memory property under other conditions, as further described herein. In one practical implementation strategy, the dominant shape memory property of shape control component 50 may define a shape of distal component segment 46 at the distally advanced location of shape control component 50 shown in Figure 1. At the retracted location of shape control component 50 shown in Figure 2, distal component segment 46 has begun to assume a shape which is defined by the subordinate shape memory property. At progressively more retracted locations of component 50, relatively more of component segment 46 will assume a shape defined by the subordinate shape memory property. Another way to understand the relationship between the respective shape memory properties is that the dominant shape memory property may define a shape of any portion of flow control component 42 within which shape control component 50 is presently positioned. Portions of flow control component 42 within which shape control component 50 is not presently positioned may have a shape defined by the subordinate shape memory property. In the example embodiments described herein, shape control component 50 includes a rest state which defines a generally linear shape. Thus, when no external deforming force is applied to shape control component 50, it may be expected to assume a generally linear configuration. Portions of flow control component 42 within which shape control component 50 is presently positioned may likewise be expected to assume a generally linear shape in response to the dominant shape memory property.

Flow control component 42 may be understood to be in a biased state in the low profile or lumen access configuration of flow managing mechanism 40, where the dominant shape memory property is determining its shape. As alluded to above, in the biased state of flow control component 42, it may have a generally linear shape. Flow control component 42 may also include a rest state, assumed when shape control component 50 has been retracted, which includes a non-linear, or curvilinear, shape. Thus, in the lumen access configuration, flow control component 42 may be generally linear to facilitate access to a body lumen, but may have a non-linear shape in the deployed configuration. It will further be understood that the biased state of flow control component 42 may include a state in which helical wire body 54 is radially condensed relative to longitudinal axis A. It will still further be understood that the rest state of flow control component 42 may include a state in which helical wire body 54 is radially expanded relative to longitudinal axis A.

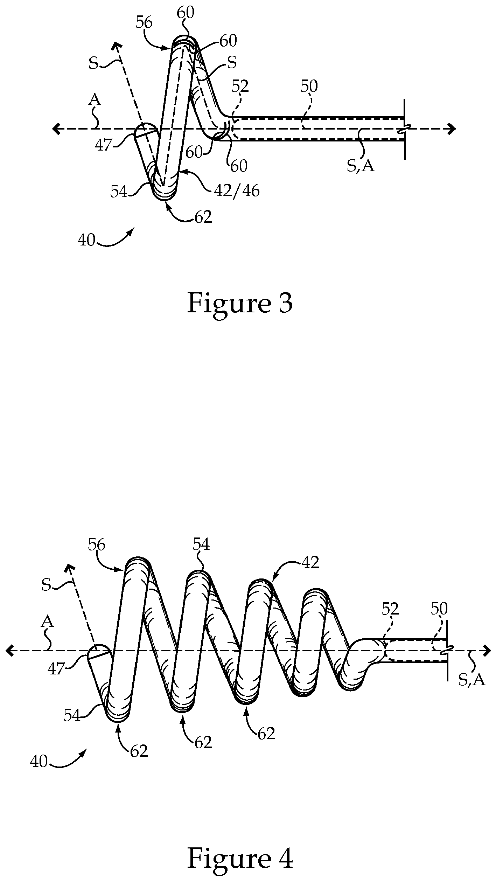

As discussed above, a shape of flow control component 42 may be changed by sliding shape control component 50 in distal to proximal or proximal to distal directions relative to flow control component 42. Referring also to Figure 3, there is shown flow managing mechanism 40 where shape control component 50 has been further retracted within flow control component 42 relative to the state shown in Figure 2. A relatively greater axial length of flow control component 42 now assumes a shape defined by the subordinate shape memory property. As mentioned above, flow control component 42 may include windings 56. Windings 56 may define a plurality of wire turns 60, corresponding to individual turns of wire body 54, about longitudinal axis S. Windings 56 may also define a plurality of wire turns about longitudinal axis A, although it may be noted that some of wire turns 60 are not positioned about longitudinal axis A in the configuration shown in Figure 3. Windings 56 may also include at least one body turn 62 about longitudinal axis A, corresponding to turns of the overall wire body 54. Wire turns 60 may thus be understood to define a minor curving shape about longitudinal axis A, which may include a spiral shape, whereas body turns 62 may be understood to define a major curving shape about longitudinal axis A, which may also include a spiral shape.

Referring also to Figure 4, there is shown flow managing mechanism 40 at a state in which shape control component 50 has been still further retracted within flow control component 42 relative to the state illustrated in Figure 3. A still greater proportion of flow control component 42 has assumed a shape defined by the subordinate shape memory property. In light of the foregoing description, it may be appreciated that flow managing mechanism 40 may have a range of deployed configurations corresponding to a range of tertiary shapes of component 42. In one practical implementation strategy, the range of deployed configurations may correspond with a range of progressively more radially expanded shapes of flow control component 42, as component 50 is progressively retracted. In other embodiments, more complex shape adjusting strategies might be used. For example, component 42 might be adapted to initially curve radially outward from axis A upon retracting component 50 a first distance, then "ball up" as component 50 is further retracted, and then assume yet another overall shape as component 50 is still further retracted. In still other embodiments, component 42 might curve back on itself in a proximal direction, back toward proximal segment 44. Each shape attainable by retracting component 50 could have a different advantageous use.

As discussed above, the shape of flow control component 42 which may be varied by sliding shape control component 50 therein may be understood as a mutable tertiary shape, and flow control component 42 may also be understood to include a fixed primary shape, and a fixed secondary shape. The terms "primary shape," "secondary shape," and "tertiary shape," are used herein in a manner analogous to similar terms used to describe a hierarchical shape of certain biomolecules such as proteins. Thus, the primary shape of flow control component 42 may be understood as a low level shape, the secondary shape may be understood as middle level shape, and the tertiary shape may be understood as a high level shape. Stated yet another way, the fixed primary shape may be a basic shape of the wire from which wire body 54 is formed, the fixed secondary shape may be understood as a shape into which the wire is wound, and the mutable tertiary shape may be understood as a shape which the wound wire body assumes under the conditions described herein.

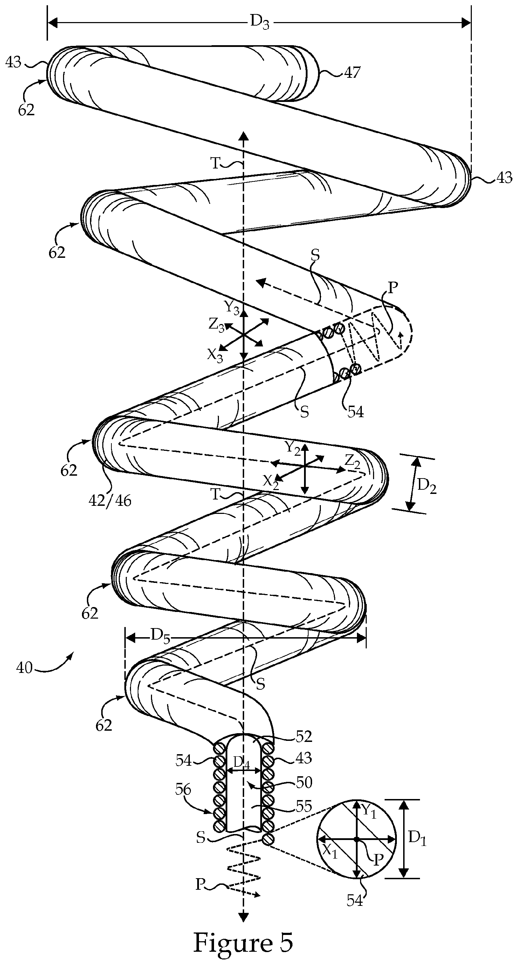

Referring now to Figure 5, there is shown flow managing mechanism 40 in a deployed configuration similar to that shown in Figure 4, and illustrating further geometric attributes associated with the primary, secondary, and tertiary shapes. As discussed above, outer wire body 54 may include a plurality of windings 56. Outer wire body 54 may further define a primary axis P which is internal to outer wire body 54. Primary axis P may include a longitudinal center axis of the wire from which outer wire body 54 is formed. The fixed primary shape of flow control component 42 may include a cross sectional shape of outer wire body 54 in two dimensions defined by primary axis P. In the illustrated embodiment, the fixed primary shape may include a rounded cross-sectional shape such as a circular shape in the plane of the page of Figure 5. The two dimensions Y₁ and X₁ defined by primary axis P are perpendicular to one another and each intersect primary axis P, and are oriented perpendicular to primary axis P. A circular fixed primary shape represents one practical implementation strategy, however, in other embodiments a non-circular shape such as an oval shape or a polygonal shape might be used, for example.

Outer wire body 54 may further define a secondary axis which includes longitudinal S. The secondary axis S may be external to outer wire body 54 and internal to windings 56. The secondary shape may include a fixed secondary shape in three dimensions defined by axis S. The fixed secondary shape may include the minor curving shape of outer wire body 54, such as a helical shape. In Figure 5, the three dimensions defined by secondary axis S are shown as dimensions X₂, Y₂ and Z₂ and the fixed secondary shape includes a helical shape with axis S comprising a helix center axis overlapping with dimension Z₂.

Outer wire body 54 may still further define a tertiary axis T. Tertiary axis T may include a geometric center axis of flow control component 42. Thus, axis T may be defined by geometric center points of the tertiary shape of flow control component 42 proceeding in a proximal to distal direction. Although axis T may intersect wire body 54 and windings 56 at certain locations, at least a majority of axis T may be external to outer wire body 54 and external to windings 56 when flow control component 42 is in the rest state, and mechanism 40 is in the low profile lumen access configuration approximately as shown in Figure 5. Referring back to Figure 1, it may be appreciated that tertiary axis T may be external to wire body 54 and internal to windings 56 when flow control component 42 is in the biased state and mechanism 40 is in the expanded or deployed configuration. In the low profile configuration, axes S, A, and T may all be overlapping.

The tertiary shape of flow control component 54 may include a shape of wire body 54 in three dimensions defined by tertiary axis T, and labeled X₃, Y₃, and Z₃ in Figure 5. The mutable tertiary shape may further include a major curving shape such as a spiral or helical shape in dimensions X₃, Y₃, and Z₃. The tertiary shape may further include a lumen occluding shape in the low profile configuration, which includes at least one turn about tertiary axis T. The at least one turn may correspond to body turns 62 described above and also shown in Figure 5. In Figure 5, the tertiary shape includes a total of five turns 62 advancing in a distal direction about tertiary axis T, however, a different number of turns may be used depending upon the application. As used herein, the term "helical" shape is intended to refer to a type of spiral shape. Thus, the tertiary shape shown in Figure 5 may be understood as a curving shape, and a spiral shape, but not a helical shape. The tertiary shape shown in Figure 5 may also be understood as having a distally expanding tapered shape.

Also shown in Figure 5 are several dimensional attributes of flow managing mechanism 40. In particular, a primary dimension D₁ corresponding to an outer diameter or wire thickness of wire body 54 is shown, and may be equal to between about 2/1000ths inches or 0.05 millimeters, and about 3/1000ths inches or 0.08 millimeters in one embodiment. A secondary dimension corresponding to an outer diameter dimension of a coil or helix into which outer wire body 54 is wound is also shown in Figure 5, and may be less than about 20/1000ths inches or 0.51 millimeters and further may be equal to between about 11/1000ths inches or 0.28 millimeters and about 18/1000ths inches or 0.46 millimeters in one embodiment. In one further embodiment, dimension D₁ may be equal to about 2/1000ths inches or 0,05 millimeters and dimension
D₂ may be equal to about 11/1000ths inches or 0,28 millimeters. In still another embodiment, dimension
D₁ may be equal to about 3/1000ths inches or 0,08 millimeters and dimension D₂ may be equal to between about 15/1000ths inches or 0.38 millimeters and about 18/1000ths inches or 0,46 millimeters.

Another dimension D₃ is shown in Figure 5 and corresponds to a mutable outer diameter dimension in a direction perpendicular to and intersecting tertiary axis T, and extending between radially outermost points defined by outer surface 43 relative to axis T. Dimension D₃ may be equal to about 118/1000ths inches or about 3 millimeters in one embodiment. From a maximum width equal to D₃, the tertiary shape depicted in Figure 5 may taper in a proximal direction to another mutable outer diameter dimension D₅ of about 79/1000ths inches or 2 millimeters, approximately halfway between the body turns 62 indicated first and second from the bottom in Figure 5. In one further embodiment, D₃ may be equal to less than about 20/1000ths inches or 0,51 millimeters in the lumen access configuration, and greater than about 20/1000ths inches and less than about 118/1 000ths inches or about 3 millimeters in the deployed configuration. Still another dimension D₄ is shown in Figure 5, and represents an outer diameter or wire thickness of wire body 55. D₄ may be equal to between about 5/1000ths inches or 0.13 millimeters and about 7/1000ths inches or about 0.18 millimeters, in one embodiment. In one further example embodiment, D₄ may be equal to at least about 33%, and may be equal to 40% or greater, of D₂, which corresponds to a diameter of control lumen 51.

As used herein, "about" 2 millimeters may be understood as equal to between 1.5 and 2.4 millimeters. "About" 2/1000ths inches may be understood to be between 1.5/1000ths inches and 2.4/1000ths inches, and so on. It should further be appreciated that the mutable dimensions will typically vary depending upon where shape control component 50 is axially positioned within flow control component 42. It may thus be appreciated that a range of length, width, height, shape, etc., of flow control component 42 may be available, and a clinician manipulating flow managing mechanism 40 as further described herein may choose to utilize a shape and/or dimensions of flow control component 42 which need not be specifically determined until the associated procedure is being performed. This faculty is contemplated to provide improvements over many conventional designs in which shape and dimensions do not have the essentially infinite adjustability attendant to the present disclosure.

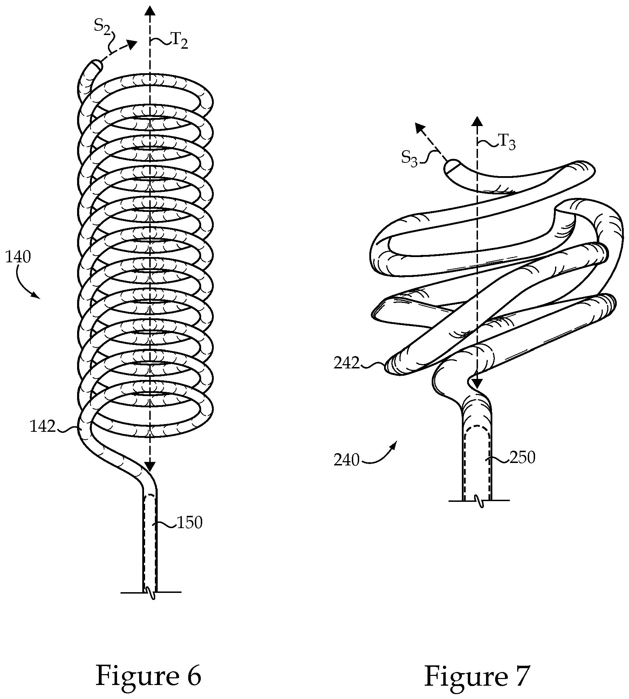

Turning now to Figure 6, there is shown a flow managing mechanism 140 according to another embodiment. Flow managing mechanism 140 includes an elongate flow control component 142 which may include a helical wire body similar to flow control component 42 described above. Flow managing mechanism 140 may further include an elongate shape control component 150 which may include a non-helical wire body also similar to that described above. Elongate flow control component 142 may include a fixed primary shape, a fixed secondary shape, and a mutable tertiary shape. Flow control component 142 is shown as it might appear in a deployed configuration of flow managing mechanism 140, in which a secondary axis S₂ curves about a tertiary axis T₂, for example in a helical pattern. Flow managing mechanism 140 may be similar in many respects to flow managing mechanism 40 described above, but rather than a tapering spiral tertiary shape, flow control component 142 may be configured to assume a uniform diameter helical tertiary shape.

Turning to Figure 7, there is shown a flow managing mechanism 240 according to yet another embodiment, and including a flow control component 242 and a shape control component 250. Flow managing mechanism 240 is also similar in many respects to the embodiments described above, but includes a complex tertiary shape. Flow control component 242 defines a secondary axis S₃, and a tertiary axis T₃. In some instances, a complex shape such as the tertiary shape of mechanism 240 may be useful for embolization of relatively larger blood vessels, such as those larger than about 5 millimeters, and those larger than about 10 millimeters. It may be noted that the tertiary shapes of the embodiment of Figure 7 includes a plurality of turns about tertiary axis T₃. In still other embodiments, diamond-shaped or spherical tertiary shapes might be used.

A variety of materials and manufacturing techniques may be used in constructing intraluminal treatment apparatuses, wire guides, and components thereof in accordance with the present disclosure. Techniques are known whereby wires and wire coils of metallic materials such as stainless steel, Nitinol®, and other metallic materials may be treated to impart a range of shape memory properties, and a wide variety of shapes assumed when the wires and wire coils are in a rest state. A variety of techniques are also known whereby the dominant shape memory property associated with shape control components 50, 150, 250, and the subordinate shape memory properties associated with flow control components 42, 142, 242, may be established. For example, shape control components 50, 150, 250 might be formed from a metallic material having a relatively greater stiffness than material from which flow control components 42, 142, and 242 may be formed. Processing techniques such as heat treating may be used whereby wire stiffness is tailored to achieve a dominant versus subordinate shape memory property. In still further examples, differing gauge and/or shape of the wire used to manufacture shape control components 50, 150, 250 versus flow control components 42, 142, and 242, could impart the desired differing shape memory properties.

### Industrial Applicability

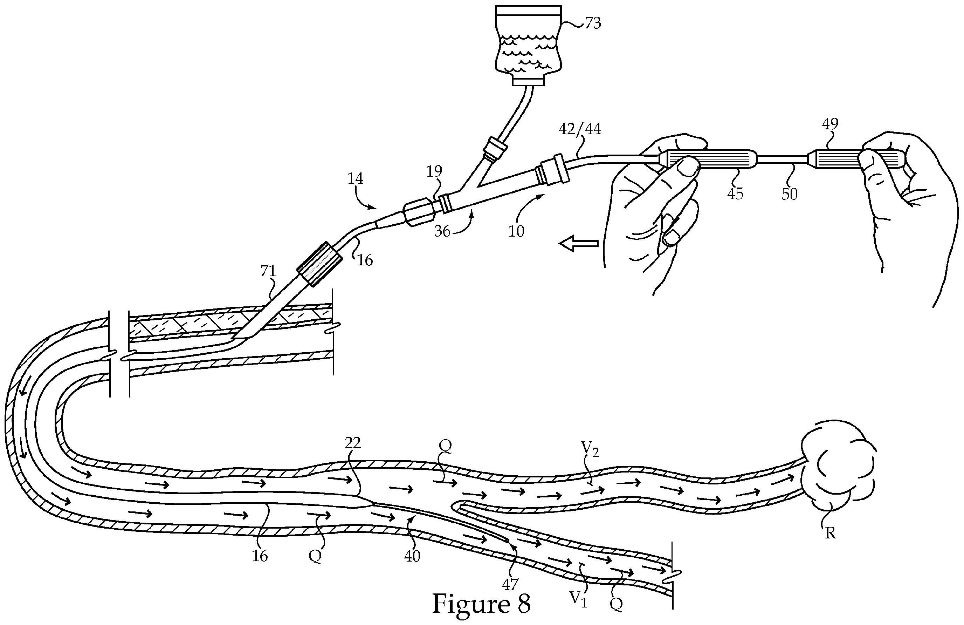

Referring to Figure 8, there is shown intraluminal treatment apparatus 10 as it might appear at one stage of a treatment procedure according to the present disclosure. It may be recalled that fluid supply mechanism 14 may include a fitting or manifold 19 which is configured to connect with an adapter such as Y-fitting 36, approximately as shown in Figure 8. A fluid supply 73 is connected with Y-fitting 36 to enable the supplying of a treatment fluid such as a chemotherapeutic agent to a body lumen of a patient, as further described herein. Apparatus 10 is also shown as it might appear having been placed by way of percutaneous access to a patient's cardiovascular system. Access for certain procedures contemplated herein may include femoral or brachial access, for example for treating a kidney tumor, however the present disclosure is not thereby limited. An introducer sheath 71 passes through the patient's skin, and elongate catheter body 16 extends through the vasculature to a point at which catheter tip 22 is positioned upstream of each of a first vascular lumen V₁, and a second vascular lumen V₂ in fluid communication with vascular lumen V₁.

In Figure 8, arrows Q denote an approximate anterograde direction of blood flow within the patient's vasculature. It may be noted that blood is flowing into vascular lumen V₁ and also into vascular lumen V₂. A treatment site such as a tumor R is downstream of elongate catheter body 16 and supplied with blood via vascular lumen V₂. Flow managing mechanism 40 is located distally of catheter tip 22 and extends into vascular lumen V₁. As mentioned above, apparatus 10 includes an assembly configuration of mechanism 40 and mechanism 14. In the assembly configuration shown in Figure 8, mechanism 40 may be positioned in passage/device lumen 26, and each of components 42 and 50 extends out of catheter body 16 from both proximal passage end 32 and distal passage end 34.

In one embodiment, flow managing mechanism 40 may be used as a wire guide to assist in positioning fluid supply mechanism 14 as desired within the patient's vasculature. For such purposes, distal ends of both components 42 and 50 might be relatively flexible to enable manipulation through tortuous vascular passages. In other embodiments, a conventional wire guide might be positioned at a location of interest, and then elongate catheter body 16 guided in an over-the-wire manner by way of the conventional wire guide. The conventional wire guide might then be removed, and flow managing mechanism 40 passed through elongate catheter body 16 to reach the state approximately as depicted in Figure 8. Where a conventional wire guide is included in package 12, a clinician could select either option. Those skilled in the art will appreciate that a variety of different placement procedures might be employed, and the present disclosure is not limited to any particular strategy. Moreover, mechanism 40 might also serve as a wire guide for still other procedures not involving the use of mechanism 14 at all.

It will be recalled that shape control component 50 is not attached to flow control component 42 and is freely slidable within flow control component 42 to adjust flow managing mechanism 40 between a lumen access configuration and a deployed configuration. With flow managing mechanism 40 positioned approximately as shown in Figure 8, shape control component 50 may be slid relative to flow control component 42 between a first location at which the dominant shape memory property of shape control component 50 imparts a lumen access configuration to flow managing mechanism 40, and a second location at which the subordinate shape memory property of flow managing mechanism 42 imparts a deployed configuration to flow managing mechanism 40. In Figure 8, apparatus 10 is shown equipped with a first manipulation handle 45 coupled with flow control component 42, and a second manipulation handle coupled with shape control component 50. Sliding shape control component 50 relative to flow control component 42 might take place by pulling shape control component 50 in a distal to proximal direction. Alternatively, or in combination with pulling shape control component 50, flow control component 42 may be pushed in a proximal to distal direction to advance flow control component 42 beyond distal tip 52 of shape control component 50. One practical implementation strategy includes holding shape control component 50 relatively stationary, and pushing flow control component 42 in the proximal to distal direction. This particular technique is believed to assist a clinician in positioning flow control component 42 at a desired location upon deployment, with the assistance of radiography. Regardless of whether pushing, pulling, or a combination thereof is used, shape control component 50 may be repositioned such that its dominant shape memory property no longer defines the tertiary shape of flow control component 42, and instead the tertiary shape is defined by the subordinate shape memory property. A clinician might also be expected to perform adjustments to flow managing mechanism 40 in vivo until a desired placement, orientation and tertiary shape is achieved. Thus, a range of tertiary shapes up to a fully deployed configuration might be used. Flow control component may be somewhat "oversized" relative to vascular lumens within which it is to be used.

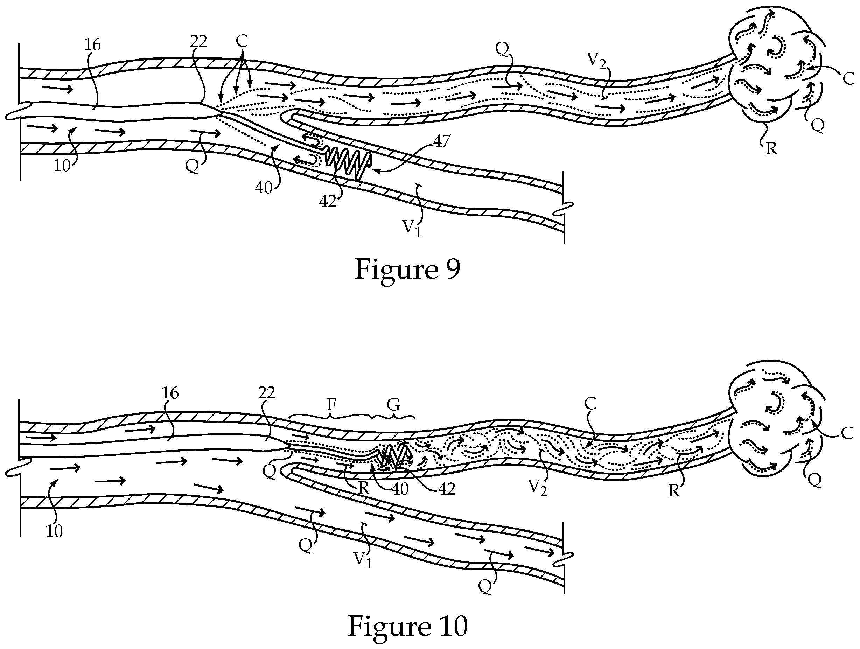

Referring also to Figure 9, there is shown apparatus 10 as it might appear where flow managing mechanism 40 has been adjusted from a low profile lumen access configuration to an expanded, deployed configuration within vascular lumen V₁. At the stage depicted in Figure 9, flow control component 42 occludes vascular lumen V₁ to block or substantially block blood flow into vascular lumen V₁. In Figure 9, a treatment fluid such as a chemotherapeutic agent has been supplied into the patient's vasculature upstream of each of vascular lumen V₁ and vascular lumen V₂. The treatment fluid is denoted via reference numeral C. As a result of occluding vascular lumen V₁, treatment fluid is supplied to vascular lumen V₂, but is not supplied or substantially limited in supply to vascular lumen V₁. It may also be noted from Figure 9 that treatment fluid C has been carried into tumor R via the flow of blood through vascular lumen V₂.

A typical chemotherapeutic treatment such as a treatment of a tumor within a kidney may be a relatively short procedure, where the treatment fluid is supplied for a time less than thirty minutes. To conclude treatment, the clinician will typically take appropriate steps to cease supplying the treatment fluid, and remove whatever devices from the patient which can be removed. The vascular lumen through which a chemotherapeutic agent is delivered in certain of these procedures may be quite small, for example less than about five millimeters. In some instances, arteries supplying fluid to a kidney tumor or other treatment site may be less than about two millimeters in internal diameter. State of the art techniques for treatment of tumors in these and other scenarios often included leaving embolization mechanisms in place within the patient, or chemotherapy was simply administered without the benefits of embolization. It was also common in many prior techniques for fibers or the like attached to an embolization mechanism to be used to intentionally promote formation of thrombus material within an embolized vascular lumen. It has been discovered that such fibers or other embolus-promoting attachments are not needed in certain procedures as contemplated herein, and the exposed outer surface 53 of component 42 can interact with blood flow to successfully embolize a vascular lumen, even where relatively small spaces between and among parts of wire body 54 exist in the expanded configuration. This may be due at least in part to the relatively slurry-like behavior of blood at the small scales contemplated herein. In any event, flow control component may be free of such fibers so that thrombus formation does not substantially occur. In one embodiment, flow control component 42 may also be coated with a material such as Heparin to inhibit formation of thrombus material.

In further contrast to certain known techniques, the present disclosure contemplates retrieving an embolization mechanism such as flow managing mechanism 40 upon concluding the treatment procedure. Flow control component 42 is not decoupled from other parts of apparatus 10, and thus may be readily retrieved. To this end, flow managing mechanism 42 may be removed from vascular lumen V₁ after ceasing supplying the treatment fluid at least in part by radially condensing flow control component 42. In particular, radially condensing flow control component 42 may include sliding shape control component 50 relative to flow control component 42 back toward the distally advanced location of shape control component 50. Radially condensing flow control component 42 might take place by pushing shape control component 50 in a proximal to distal direction, pulling flow control component 42 in a distal to proximal direction, or a combination of the two. In still other embodiments, a sheath may be used to collapse or otherwise radially condense flow managing mechanism 40. Elongate catheter body 16 might also be configured such that flow managing mechanism 40 could be pulled inside or partially inside for similar purposes. Once flow managing mechanism 40 has been partially or completely returned to a low profile configuration, blood flow may be restored to vascular lumen V₁, apparatus 10 removed from the patient, and other appropriate post-procedural activities undertaken.

Turning now to Figure 10, there is shown an intraluminal treatment apparatus 10 positioned within the vasculature of a patient during an intraluminal treatment procedure having certain similarities with the procedure described above, but several important differences. Instead of occluding a vascular lumen via flow control component 42, in the application depicted in Figure 10 flow control component 42 is used to manage flow of fluid within vascular lumen V₂ to improve dispersion and mixing of treatment fluid such as a chemotherapeutic agent Q within the blood flowing through vascular lumen V₂. Mechanism 10 may be only partially deployed in the procedure shown in Figure 10, to avoid completely occluding lumen V₂.

Certain tumors may have a relatively complex vasculature. Numerous small blood vessels may form a fairly chaotic blood supply network within tumor tissue as compared to normal body tissues. It is believed that this relatively un- ordered vascular structure can render the tumor relatively insensitive to certain chemotherapeutic techniques. For example, where a treatment fluid is supplied by way of blood flow to the vasculature of a tumor, relatively poor mixing of the chemotherapeutic agent in the blood can result in certain parts of the tumor receiving an inordinate proportion of the chemotherapeutic agent, while other parts of the tumor receive little, if any. This phenomenon is believed to be due at least in part to a relatively laminar flow of blood and thus treatment fluid through a vascular lumen upstream of the treatment site. When the blood arrives at the tumor, certain parts of the tumor vasculature may disproportionately receive the chemotherapeutic agent. This potentially reduces the effectiveness of the chemotherapy procedure, or may require excessive quantities of chemotherapeutic agent, or long treatment durations.

In Figure 10, a first zone F of vascular lumen V₂ is shown which extends approximately from catheter tip 22 to a deployed portion of flow control component 42. In the embodiment shown, flow control component 42 has been adjusted via sliding a shape control component (not shown) therein in a manner similar to that described above such that the flow control component 42 assumes an expanded tertiary shape, body possibly not fully expanded as alluded to above. In other embodiments, substantially different tertiary shapes might be used. It may be noted that the flow of blood and treatment fluid in zone F is relatively uniform and, hence, relatively laminar. A second zone G of vascular lumen V₂ is also shown, and corresponds approximately to a portion of vascular lumen within which flow control component 42 has the deployed, tertiary shape. It may be noted that the flow of blood and treatment fluid in zone G is relatively non-uniform, and the blood and treatment fluid may flow over, around, and through flow control component 42. As a result, relatively laminar fluid flow through zone F changes to relatively turbulent flow through zone G, and an improved mixing of treatment fluid within the blood in vascular lumen V₂ is achieved.

## Claims

1. An intraluminal treatment apparatus (10) comprising:
a fluid supply mechanism (14) including an elongate catheter body (16) having a profiled catheter tip (22) defining a fluid outlet (35), the elongate catheter body being configured to supply a treatment fluid to a body lumen of a patient, and at least one longitudinally extending passage (24, 26) connecting with the fluid outlet; and
a flow managing mechanism (40) positionable within the at least one passage for controlling said flow of the treatment fluid within the body lumen, and including an elongate flow control component (42) defining a longitudinal axis (S) and having a subordinate shape memory property, and an elongate shape control component (50) positioned within the flow control component and slidable relative to the flow control component, the shape control component having a dominant shape memory property;
the flow control component further having a fixed primary shape, a fixed secondary shape, and a mutable tertiary shape, and
the flow managing mechanism having a lumen access configuration in which the shape control component is positioned at a first axial location within the flow control component and the dominant shape memory property defines the tertiary shape, and a deployed configuration in which the shape control component is positioned at a second axial location and the subordinate shape memory property defines the tertiary shape.

2. The intraluminal treatment apparatus of claim 1 wherein:
the flow control component includes a proximal component segment (44), and a distal component segment (46) having the mutable tertiary shape;
the first axial location includes a distally advanced location within the proximal component segment and the second axial location includes a retracted location; and
the distal component segment further having a biased state in the lumen access configuration, and a rest state in the deployed configuration.

3. The intraluminal treatment apparatus of claim 2 wherein the distal component segment includes an exposed outer surface (43) defining a fixed outer diameter dimension through the longitudinal axis which is equal to less than about 20/1000ths inches or 0,51 millimeters, and defining a mutable outer diameter dimension (D5) which is equal to less than about 20/1000ths inches or 0,51 mm, in the lumen access configuration, and equal to greater than about 20/1000ths inches or 0,51 millimeters and less than about 118/1000ths inches or 3 millimeters, in the deployed configuration.

4. The intraluminal treatment apparatus of claim 2 wherein the at least one passage includes a passage length extending between a proximal passage end (32) and a distal passage end (34), and each of the flow control component and the shape control component includes an axial component length greater than the passage length.

5. The intraluminal treatment apparatus of claim 4 wherein:
the at least one passage further includes a longitudinally extending fluid supply lumen, and a longitudinally extending device lumen which includes the proximal passage end and the distal passage end; and
the intraluminal treatment apparatus includes an assembly configuration where the flow managing mechanism is positioned within the device lumen and each of the shape control component and the flow control component extends from the proximal passage end and the distal passage end, and the distal component segment being located distally to the profiled catheter tip in the assembly configuration.

6. The intraluminal treatment apparatus of claim 2 wherein the distal component segment includes a wire body (54) having a plurality of windings (56), the wire body defining a primary axis (P) which is internal to the wire body, and a secondary axis (S, S2, S3) which includes the longitudinal axis and is external to the wire body and internal to the plurality of windings.

7. The intraluminal treatment apparatus of claim 6 wherein the wire body defines a tertiary axis (T, T2, T3), the tertiary axis being external to the wire body and external to the plurality of windings in the deployed configuration, and being external to the wire body and internal to the plurality of windings in the lumen access configuration, and wherein the distal component segment includes at least one turn about the tertiary axis in the deployed configuration.

8. The intraluminal treatment apparatus of claim 1 wherein:
the shape control component includes a non-helical wire body (55) defining a second longitudinal axis, and the flow control component includes a helical wire body (54) having an exposed outer wire body surface (43), an inner wire body surface (48), and a plurality of windings (56) which include each of the outer and inner wire body surfaces;
the flow control component including a biased state in the lumen access configuration and a rest state in the deployed configuration; and
the secondary shape includes a minor curving shape about the second longitudinal axis and the tertiary shape includes a major curving shape about the second longitudinal axis, each of the minor curving shape and the major curving shape being defined by the plurality of windings.

## Patentansprüche

1. Intraluminale Behandlungsvorrichtung (10), umfassend:
einen Flüssigkeitszuführungsmechanismus (14) mit einem länglichen Katheterkörper (16), der eine profilierte Katheterspitze (22) aufweist, die einen Flüssigkeitsauslass (35) definiert, wobei der längliche Katheterkörper zum Zuführen einer Behandlungsflüssigkeit zu einem Körperlumen eines Patienten ausgelegt ist, und zumindest einen sich längs erstreckenden Durchgang (24, 26), der mit dem Flüssigkeitsauslass verbunden ist; und
einen Durchflussmanagementmechanismus (40), der zur Steuerung des Durchflusses der Behandlungsflüssigkeit innerhalb des Körperlumens in dem zumindest einen Durchgang positionierbar ist, und umfassend eine längliche Durchflusskontrollkomponente (42), die eine Längsachse (S) definiert und eine untergeordnete Formgedächtniseigenschaft aufweist, und eine längliche Formkontrollkomponente (50), die innerhalb der Durchflusskontrollkomponente angeordnet ist und bezüglich der Durchflusskontrollkomponente verschiebbar ist, wobei die Formkontrollkomponente eine dominante Formgedächtniseigenschaft aufweist;
wobei die Durchflusskontrollkomponente ferner eine feste primäre Gestalt, eine feste sekundäre Gestalt und eine veränderliche tertiäre Gestalt aufweist, und wobei der Durchflussmanagementmechanismus eine Lumenzugangskonfiguration, in der die Formkontrollkomponente an einer ersten axialen Position innerhalb der Durchflusskontrollkomponente angeordnet ist und die dominante Formgedächtniseigenschaft die tertiäre Gestalt definiert, und eine eingesetzte Konfiguration aufweist, in der die Formkontrollkomponente an einer zweiten axialen Position angeordnet ist und die untergeordnete Formgedächtniseigenschaft die tertiäre Gestalt definiert.

2. Intraluminale Behandlungsvorrichtung nach Anspruch 1, wobei:
die Durchflusskontrollkomponente ein proximales Komponentensegment (44) und ein distales Komponentensegment (46) mit der veränderlichen tertiären Gestalt aufweist;
die erste axiale Position eine distal vorgeschobene Position innerhalb des proximalen Komponentensegments aufweist und die zweite axiale Position eine zurückgezogene Position aufweist; und
das distale Komponentensegment ferner einen vorgespannten Zustand in der Lumenzugangskonfiguration und einen Ruhezustand in der eingesetzten Konfiguration aufweist.

3. Intraluminale Behandlungsvorrichtung nach Anspruch 2, wobei das distale Komponentensegment eine freiliegende Außenfläche (43) aufweist, die eine feste Außendurchmesserdimension durch die Längsachse definiert, die kleiner ist als ungefähr 20/1000stel Zoll oder 0,51 Millimeter, und eine veränderliche Außendurchmesserdimension (D5) definiert, die kleiner ist als ungefähr 20/1000stel Zoll oder 0,51 mm, in der Lumenzugangskonfiguration, und größer ist als ungefähr 20/1000stel Zoll oder 0,51 Millimeter und kleiner als ungefähr 118/1000stel Zoll oder 3 Millimeter in der eingesetzten Konfiguration.

4. Intraluminale Behandlungsvorrichtung nach Anspruch 2, wobei der zumindest eine Durchgang eine Durchgangslänge aufweist, die sich zwischen einem proximalen Durchgangsende (32) und einem distalen Durchgangsende (34) erstreckt, und die Durchflusskontrollkomponente und die Formkontrollkomponente jeweils eine axiale Komponentenlänge aufweisen, die größer als die Durchgangslänge ist.

5. Intraluminale Behandlungsvorrichtung nach Anspruch 4, wobei:
der zumindest eine Durchgang ferner ein sich längs erstreckendes Flüssigkeitszuführungslumen und ein sich längs erstreckendes Vorrichtungslumen aufweist, welches das proximale Durchgangsende und das distale Durchgangsende aufweist; und
die intraluminale Behandlungsvorrichtung eine Montagekonfiguration aufweist, in welcher der Durchflussmanagementmechanismus innerhalb des Vorrichtungslumens angeordnet ist und sich die Formkontrollkomponente und die Durchflusskontrollkomponente jeweils vom proximalen Durchgangsende und vom distalen Durchgangsende erstrecken, und das distale Komponentensegment distal zur profilierten Katheterspitze in der Montagekonfiguration angeordnet ist.

6. Intraluminale Behandlungsvorrichtung nach Anspruch 2, wobei das distale Komponentensegment einen Drahtkörper (54) mit einer Mehrzahl von Windungen (56) aufweist, wobei der Drahtkörper eine primäre Achse (P), die innerhalb des Drahtkörpers liegt, und eine sekundäre Achse (S, S2, S3), welche die Längsachse beinhaltet und außerhalb des Drahtkörpers und innerhalb der Mehrzahl von Windungen liegt, definiert.

7. Intraluminale Behandlungsvorrichtung nach Anspruch 6, wobei der Drahtkörper eine tertiäre Achse (T, T2, T3) definiert, wobei die tertiäre Achse außerhalb des Drahtkörpers und außerhalb der Mehrzahl von Windungen in der eingesetzten Konfiguration liegt, und außerhalb des Drahtkörpers und innerhalb der Mehrzahl von Windungen in der Lumenzugangskonfiguration liegt, und wobei das distale Komponentensegment zumindest eine Windung um die tertiäre Achse in der eingesetzten Konfiguration aufweist.

8. Intraluminale Behandlungsvorrichtung nach Anspruch 1, wobei:
die Formkontrollkomponente einen nichtspiralförmigen Drahtkörper (55) aufweist, der eine zweite Längsachse definiert, und die Durchflusskontrollkomponente einen spiralförmigen Drahtkörper (54) aufweist, der eine freiliegende äußere Drahtkörperoberfläche (43), eine innere Drahtkörperoberfläche (48) und eine Mehrzahl von Windungen (56) aufweist, die jede der äußeren und inneren Drahtkörperoberflächen aufweisen;
die Durchflusskontrollkomponente einen vorgespannten Zustand in der Lumenzugangskonfiguration und einen Ruhezustand in der eingesetzten Konfiguration aufweist; und
die sekundäre Gestalt eine gebogene Nebengestalt um die zweite Längsachse aufweist und die tertiäre Gestalt eine gebogene Hauptgestalt um die zweite Längsachse aufweist, wobei die gebogene Nebengestalt und die gebogene Hauptgestalt jeweils von der Mehrzahl von Windungen definiert werden.

## Revendications

1. Appareil de traitement intraluminal (10) comprenant :
un mécanisme de fourniture de fluide (14) comportant un corps de cathéter allongé (16) ayant une pointe de cathéter profilée (22) définissant une sortie de fluide (35), le corps de cathéter allongé étant configuré pour fournir un fluide de traitement à une lumière corporelle d'un patient, et au moins un passage s'étendant longitudinalement (24, 26) se raccordant à la sortie de fluide ; et
un mécanisme de gestion d'écoulement (40) positionnable à l'intérieur de l'au moins un passage pour contrôler ledit écoulement du fluide de traitement à l'intérieur de la lumière corporelle, et comportant un composant de contrôle d'écoulement allongé (42) définissant un axe longitudinal (S) et ayant une propriété de mémoire de forme subordonnée, et un composant de contrôle de forme allongé (50) positionné à l'intérieur du composant de contrôle d'écoulement et coulissable par rapport au composant de contrôle d'écoulement, le composant de contrôle de forme ayant une propriété de mémoire de forme dominante ;
le composant de contrôle d'écoulement ayant en outre une forme primaire fixe, une forme secondaire fixe, et une forme tertiaire modifiable, et
le mécanisme de gestion d'écoulement ayant une configuration d'accès à la lumière dans laquelle le composant de contrôle de forme est positionné à un premier emplacement axial à l'intérieur du composant de contrôle d'écoulement et la propriété de mémoire de forme dominante définit la forme tertiaire, et une configuration déployée dans laquelle le composant de contrôle de forme est positionné à un deuxième emplacement axial et la propriété de mémoire de forme subordonnée définit la forme tertiaire.

2. Appareil de traitement intraluminal de la revendication 1 dans lequel :
le composant de contrôle d'écoulement comporte un segment de composant proximal (44), et un segment de composant distal (46) ayant la forme tertiaire modifiable ;
le premier emplacement axial comporte un emplacement avancé distalement à l'intérieur du segment de composant proximal et le deuxième emplacement axial comporte un emplacement rétracté ; et
le segment de composant distal ayant en outre un état déformé dans la configuration d'accès à la lumière, et un état de repos dans la configuration déployée.

3. Appareil de traitement intraluminal de la revendication 2 dans lequel le segment de composant distal comporte une surface externe exposée (43) définissant une dimension diamétrale externe fixe à travers l'axe longitudinal qui est égale ou inférieure à environ 20/1000^{e} pouce ou 0,51 millimètre, et définissant une dimension diamétrale externe modifiable (D5) qui est égale ou inférieure à environ 20/1000^{e} pouce ou 0,51 millimètre, dans la configuration d'accès à la lumière, et égale ou supérieure à environ 20/1000^{e} pouce ou 0,51 millimètre et inférieure à environ 118/1000^{e} pouce ou 3 millimètres, dans la configuration déployée.

4. Appareil de traitement intraluminal de la revendication 2 dans lequel l'au moins un passage comporte une longueur de passage s'étendant entre une extrémité de passage proximale (32) et une extrémité de passage distale (34), et le composant de contrôle d'écoulement et le composant de contrôle de forme comportent chacun une longueur de composant axiale supérieure à la longueur de passage.

5. Appareil de traitement intraluminal de la revendication 4 dans lequel :
l'au moins un passage comporte en outre une lumière de fourniture de fluide s'étendant longitudinalement, et une lumière de dispositif s'étendant longitudinalement qui comporte l'extrémité de passage proximale et l'extrémité de passage distale ; et
l'appareil de traitement intraluminal comporte une configuration d'assemblage où le mécanisme de gestion d'écoulement est positionné à l'intérieur de la lumière de dispositif, et le composant de contrôle de forme et le composant de contrôle d'écoulement s'étendent chacun de l'extrémité de passage proximale à l'extrémité de passage distale, et le segment de composant distal étant situé distalement par rapport à la pointe de cathéter profilée dans la configuration d'assemblage.

6. Appareil de traitement intraluminal de la revendication 2 dans lequel le segment de composant distal comporte un corps de fil (54) ayant une pluralité d'enroulements (56), le corps de fil définissant un axe primaire (P) qui est interne au corps de fil, et un axe secondaire (S, S2, S3) qui comporte l'axe longitudinal et est externe au corps de fil et interne à la pluralité d'enroulements.

7. Appareil de traitement intraluminal de la revendication 6 dans lequel le corps de fil définit un axe tertiaire (T, T2, T3), l'axe tertiaire étant externe au corps de fil et externe à la pluralité d'enroulements dans la configuration déployée, et étant externe au corps de fil et interne à la pluralité d'enroulements dans la configuration d'accès à la lumière, et dans lequel le segment de composant distal comporte au moins un tour autour de l'axe tertiaire dans la configuration déployée.

8. Appareil de traitement intraluminal de la revendication 1 dans lequel :
le composant de contrôle de forme comporte un corps de fil non hélicoïdal (55) définissant un deuxième axe longitudinal, et le composant de contrôle d'écoulement comporte un corps de fil hélicoïdal (54) ayant une surface de corps de fil externe exposée (43), une surface de corps de fil interne (48), et une pluralité d'enroulements (56) qui comportent chacune des surfaces de corps de fil externe et interne ;
le composant de contrôle d'écoulement comportant un état déformé dans la configuration d'accès à la lumière et un état de repos dans la configuration déployée ; et
la forme secondaire comporte une forme de courbure mineure autour du deuxième axe longitudinal et la forme tertiaire comporte une forme de courbure majeure autour du deuxième axe longitudinal, la forme de courbure mineure et la forme de courbure majeure étant chacune définies par la pluralité d'enroulements.
